# EUROPÄISCHE PATENTANMELDUNG

(11) **EP 1 559 383 A2**
(43) Veröffentlichungstag der Anmeldung: **03.08.2005**
(21) Anmeldenummer: 04026499.6
(22) Anmeldetag: 09.11.2004
(51) Int. Cl.: A61F 2/28, A61F 2/36, A61F 2/78, A61B 17/78

(54) **Set zur Erstellung eines transkutanen Implantates**

(30) Priorität: 30.01.2004 DE 102004006065
(71) Anmelder: ESKA Implants GmbH & Co., 23556 Lübeck (DE)
(72) Erfinder: Grundei, Hans, Dr., 23558 Lübeck (DE)
(74) Vertreter: Fuchs Mehler Weiss & Fritzsche

(57) **Zusammenfassung**

Es wird ein Set zur Erstellung eines transkutanen Implantates beschrieben. Dieses weist auf:
- einen intrakorporal mit seinem proximalen Stielteil (2) in einem Femurstumpf (3) verankerbaren Adapter (1) für ein exoprothetisches Standardteil (7), wobei das Stielteil (2) zumindest teilweise mit einer offenmaschigen dreidimensionalen Raumnetzstruktur (4) bedeckt ist und an seinem distalen Ende (5) mit einer Koppelungseinrichtung (6) für das exoprothetische Standardteil (7) versehen ist.

Eine weitere Komponente ist ein mit dem proximalen Ende (8) des Adapters (1) verbindbarer Verriegelungsaufsatz (9) zur Vorbereitung des Implantates als Gelenkkugelersatzimplantat.

Alternativ kann für eine Frakturstabilisierung als weitere Komponente ein mit dem proximalen Ende (8) des Adapters (1) verbindbarer stielförmiger Aufsatz zur Frakturstabilisation vorgesehen sein.

## Beschreibung

Die Erfindung betrifft ein Set zur Erstellung eines transkutanen Implantates.

Sie geht dabei von einem Adapter für ein exoprothetisches Standardteil gemäß der DE-C-198 26 638 aus. Dieser Adapter weist ein intrakorporal mit seinem proximalen Stielteil in einem Femurstumpf verankerbaren Adapter für ein exoprothetisches Standardteil auf, wobei das Stielteil zumindest teilweise mit einer offenmaschigen, dreidimensionalen Raumnetzstruktur bedeckt ist und an seinem distalen Ende mit einer Koppelungseinrichtung für das exoprothetische Standardteil versehen ist.

Dieser Adapter hat sich seit einiger Zeit in der Praxis bewährt. Der Adapter verleiht in Verbindung mit einer daran angekoppelten Beinprothese, einem damit versorgten Patienten eine ungeahnte Osteoperzeption. Damit versorgte Patienten zeigen ein praktisch natürliches Laufbild, wobei dieses selbstverständlich noch abhängig ist von den übrigen Teilen der Prothese, also insbesondere des Kniegelenkes und des Fußgelenkes.

Bei allen Vorzügen können jedoch auch Probleme auftreten. Da ist zum einen eine potenzielle Verkeimung der Durchtrittsstelle des Adapters durch den Oberschenkelstumpf zu nennen. Dieses Problem ist aber beispielsweise gelöst durch ein subkutanes, intramuskuläres Lager gemäß der nicht vorveröffentlichten DE-A-103 53 400. Zum anderen ist zu nennen die Gefahr einer Fraktur des Oberschenkelstumpfes, sobald extrakorporal nicht eine Sollbruchstelle in mindestens einem Protheseteil vorgesehen ist, wie dies in der DE-A-100 40 617 vorgeschlagen wird. Auch ein Versagen einer Sollbruchstelle bei einem ungünstigen Sturz kann verheerende Folgen für den Patienten nach sich ziehen, namentlich eine Fraktur des Femurs im Schenkelhalsbereich. Ein drittes Problem entsteht dann, wenn der Patient zwar mit einem erwähnten Adapter und einer ganzen Beinprothese gut versorgt ist, sich aber im Bereich des natürlichen Hüftgelenkskopfes am Femur Verschleißerscheinungen auftreten. Eine Versorgung des Patienten mit herkömmlichen Hüftstielendoprothesen scheidet aus, da sich in dem intramedullären Kanal im Femurstumpf bereits der Adapter befindet und so dem Hüftstiel kein ausreichender Raum mehr zur Verfügung steht. Vor diesem Hintergrund ist es die Aufgabe der vorliegenden Erfindung, die beiden vorerwähnten Probleme zu lösen.

Hinsichtlich der Problematik einer Femurfraktur wird die Aufgabe gelöst durch ein Set der eingangs erwähnten Art mit den Merkmalen des Anspruchs 1. Dieses ist weitergebildet durch die Unteransprüche 2 bis 5. Hinsichtlich des Problems der Versorgung des Patienten mit einer künstlichen Gelenkkugel wird die Aufgabe gelöst durch ein Set mit den Merkmalen des unabhängigen Anspruchs 6, welches durch die Unteransprüche 7 bis 14 weitergebildet wird.

Der erste Lösungsvorschlag sieht zusätzlich zu dem bereits erwähnten Adapter als Teil des Sets einen mit dem proximalen Ende des Adapters verbindbaren stielförmigen Aufsatz zur Frakturstabilisation vor. Dieses Aufsatzimplantat ist besonders bevorzugt ein hülsenförmiger Armierungskörper, der von oben, also von cranial her durch eine in die Kortikalis eingebrachte Öffnung neben dem Trochanter major in einen entsprechend ausgeräumten Kanal gesetzt wird und mit dem proximalen Ende des Adapters verbunden wird. Die Verbindung kann beispielsweise und vorzugsweise erfolgen durch eine konische Klemmverbindung. Hierzu weist beispielsweise die Hülse an ihrem unteren Ende einen konischen Steckzapfen auf, während der Adapter in seinem proximalen Ende eine konische Steckhülse aufweist. Steckhülse und Steckzapfen können in bekannter Weise in eine konische Klemmverbindung gebracht werden. Zusätzlich denkbar wäre eine Sicherung durch eine Schraube.

Gemäß einer alternativen Ausführungsform kann das Aufsatzimplantat ein rohrförmiger Armierungskörper sein mit einem gekrümmten Verlauf und mit einer solchen Länge, dass er von der Verbindungsstelle mit dem Adapter im Femur durch einen Knochenkanal in den Schenkelhals einführbar ist. Dieser Armierungskörper sorgt für eine zusätzliche Armierung des Schenkelhalses.

Beide vorerwähnten Armierungskörper können besonders bevorzugt aus einem zu einem Rohr geformten Gitternetzwerk bestehen, durch welches hindurch mit zunehmender Zeit Knochentrabekel der Spongiosa einwachsen.

Alternativ hierzu kann der Armierungskörper aus massivem körperverträglichem Metall bestehen und zumindest abschnittsweise eine offenmaschige, dreidimensionale Raumnetzstruktur auf seiner Oberfläche aufweisen. In diese Raumnetzstruktur und durch diese hindurch werden wiederum Knochentrabekel wachsen und für einen stabilen Halt sorgen.

Mit dem vorbeschriebenen Set gelingt es, aufgetretene Frakturen oberhalb des proximalen Endes des Adapters zu stabilisieren. Das im Folgenden beschriebene Set bietet die Möglichkeit einer Nachversorgung des Patienten mit einem Hüftgelenkskugelimplantat.

Dieses Set weist - neben dem schon beschriebenen Adapter ― einen mit dem proximalen Ende des Adapters verbindbaren Verriegelungsaufsatz zur Vorbereitung des Implantates als Gelenkkugelersatzimplantat auf. Das Verriegelungsaufsatzimplantat bietet die Möglichkeit, dass große Kräfte in das Gesamtimplantat eingeleitet werden können, wie dies auch notwendig ist, wenn das Verriegelungsaufsatzimplantat zusammen mit dem Adapter die Kräfte aus dem Hüftgelenk aufzunehmen haben, wenn das Verriegelungsaufsatzimplantat mit einer künstlichen Gelenkkugel bestückt wird.

Besonders bevorzugt besteht das Verriegelungsaufsatzimplantat aus einer von oben (also von cranial her) neben dem Trochanter major einführbaren Hülse, die mit dem proximalen Ende des Adapters verbindbar ist, mit einer ersten Durchgangsöffnung und aus einem Verriegelungsstiel, der in einen Knochenkanal entlang des Schenkelhalses des Femurs einsetzbar ist, wobei der Verriegelungsstiel durch die erste Durchgangsöffnung in der Hülse greift. Der Knochenkanal im Schenkelhals wird von lateral hergestellt, nachdem die Kortikalis in diesem Bereich geöffnet worden ist.

Die Verriegelung zwischen der Hülse und dem Verriegelungsstiel kann in einfacher Weise durch einen Pressfit erfolgen. Bevorzugter hingegen ist aber eine Ausführungsform des Sets, bei dem die erste Durchgangsöffnung der Hülse ein Innengewinde aufweist, mit dem ein Außengewinde des Verriegelungsstiels verschraubbar ist.

Damit im Falle auftretender Verschleißerscheinungen des natürlichen Hüftgelenkskopfes eine künstliche Hüftgelenkskugel mit möglichst geringem Aufwand appliziert werden kann, ist gemäß einer bevorzugten Ausführungsform vorgesehen, dass das distale Ende des Verriegelungsstiels als konischer Steckzapfen ausgebildet ist. Im Falle einer notwendig werdenden Teilresektion des natürlichen Hüftgelenkskopfes kann nach erfolgter Resektion eine künstliche Hüftgelenkskugel auf den konischen Steckzapfen des Verriegelungsstiels gesetzt werden. Hierzu muss die künstliche Hüftgelenkskugel eine entsprechend ausgebildete konische Steckhülse aufweisen.

Eine konische Verklemmung kann besonders bevorzugt auch Anwendung finden zwischen der von cranial in den Femurstumpf einführbaren Hülse und dem proximalen Ende des Adapters.

Die vorerwähnte konische Klemmverbindung kann noch zusätzlich gesichert werden durch eine Schraubverbindung zwischen einer die Hülse durchsetzenden Schraube und einer Gewindebohrung im Adapter. Hierzu ist in dem Verriegelungsstiel eine zweite Durchgangsbohrung vorgesehen, durch welche dann die Schraube greift. Diese Ausführungsform ist naturgemäß besonders stabil.

Die nachfolgend beschriebenen Ausführungsformen betreffen nun die Beschaffenheit des Verriegelungsstiels.

Dieser kann gemäß einer Ausführungsform bis auf die zweite Durchgangsöffnung, soweit vorhanden, massiv ausgebildet sein. Diese Ausführungsform bietet bei einem eventuell notwendig werdenden Rückzug den wenigsten Widerstand gegen die Entfernung des Verriegelungsstieles.

Der Verriegelungsstiel kann aber auch wenigstens teilweise als hohler Armierungskörper aus einem Gitternetzwerk gebildet sein. Hier steht die Langzeitstabilität des Verriegelungsstiels im Schenkelhals im Vordergrund. In den hohlen Armierungskörper wächst nämlich im Laufe der Zeit Knochenmaterial ein, welches für die Langzeitstabilität in situ sorgt.

Alternativ hierzu kann der Verriegelungsstiel zumindest bereichsweise mit einer offenmaschigen, dreidimensionalen Raumnetzstruktur versehen sein. Diese Ausführungsform bietet die größte Sicherheit einer Langzeitfixation, da in die Raumnetzstruktur und durch diese hindurch Knochenmaterial einorganisiert wird.

Die Erfindung wird nachfolgend anhand von Ausführungsbeispielen näher erläutert. Hierbei zeigt:
- Fig. 1: ein Set gemäß einer Ausführungsform, schematisch dargestellt als implantiert in einem Femurstumpf,
- Fig. 2: die Ansicht eines aus diesem Set erstellten Implantates gemäß Fig. 1 von lateral her gesehen,
- Fig. 3: drei Ausführungsformen des Verriegelungsstiels, und
- Fig. 4: die von cranial neben dem Trochanter major einführbare Hülse.

Nachfolgend bezeichnen gleiche Bezugszeichen dieselben Teile.

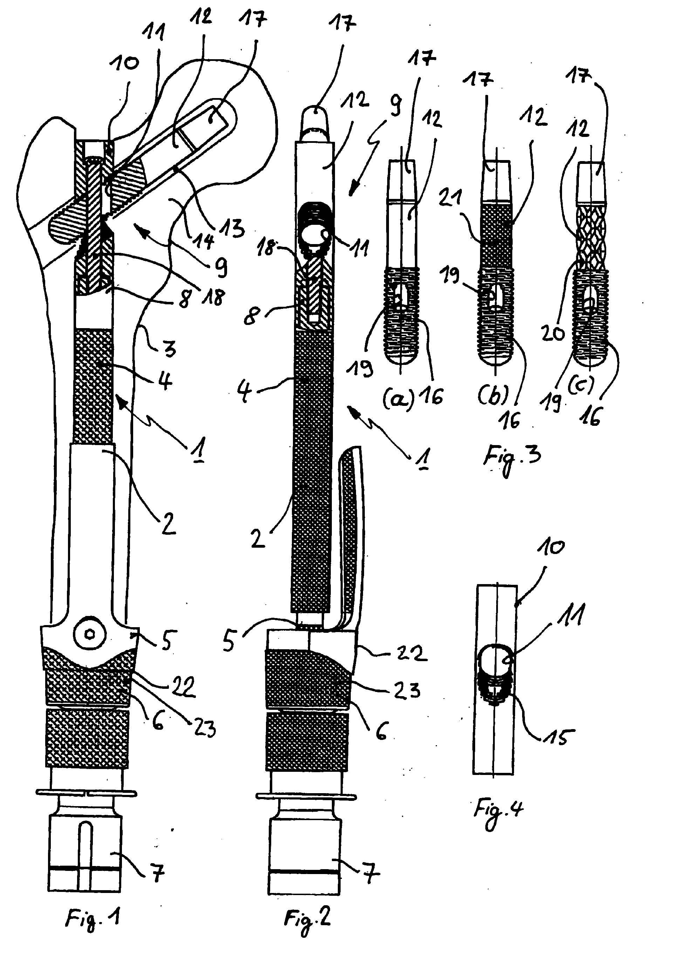

Einen ersten Überblick verschafft Fig. 1. Diese zeigt einen in einen Femurstumpf 3 eingesetzten Adapter 1, der intrakorporal mit seinem proximalen Stielteil 2 verankert ist. Der Adapter 1 weist an seinem distalen Ende 5 eine Koppelungseinrichtung 6 für ein bei 7 angedeutetes exoprothetisches Standardteil.

Für eine Langzeitfixation des Adapters 4 ist der Stielteil 2 wenigstens teilweise belegt mit einer offenmaschigen, dreidimensionalen Raumnetzstruktur 4, in welches sich Knochenmaterial einorganisiert.

Dem distalen Ende 5 des Adapters 1 schließt sich eine Buchse 22 an. Diese Buchse ist auch mit einer offenmaschigen, dreidimensionalen Raumnetzstruktur 23 belegt. Diese Raumnetzstruktur befindet sich aber schon außerhalb des Femurstumpfes 3. Die Raumnetzstruktur 23 dient dazu, dass das nach Implantation der Buchse 22 sie umgebende Bindegewebe eingranuliert, um so eine Keimsperre auszubilden (DE-A-103 53 400).

Auf dem proximalen Ende 8 des Adapters 2 ist nun vorliegend ein Verriegelungsaufsatz 9 aufgesetzt, und zwar im dargestellten Beispiel ein Verriegelungsaufsatz 9 für eine spätere Ankoppelung einer künstlichen Gelenkkugel.

Der dargestellte Verriegelungsaufsatz 9 besteht aus einer von cranial, also in Fig. 1 von oben, neben dem Trochanter major in den Femurstumpf 3 einführbare Hülse. Diese ist mit dem proximalen Ende 8 des Adapters 1 verbindbar, und zwar vorliegend - wie angedeutet - mittels eines konischen Klemmsitzes. Die Hülse 10 weist eine erste Durchgangsöffnung 11 auf.

Des Weiteren besteht der Verriegelungsaufsatz 9 aus einem Verriegelungsstiel 12. Dieser Verriegelungsstiel 12 wird nach der lateralen Eröffnung der Kortikalis in einen freigeräumten Knochenkanal 13 im Schenkelhals 14 gesetzt. Seine Position wird durch die von cranial einzusetzende Hülse 10 verriegelt.

Operativ geht man nun so vor, dass zunächst die Hülse 10 von cranial in einen freigeräumten Knochenkanal gesetzt wird und sodann von lateral her der Verriegelungsstiel 12 in den Knochenkanal 13 gesetzt wird, wobei der Verriegelungsstiel 12 dabei durch die erste Durchgangsöffnung 11 in der Hülse 10 greift.

In der dargestellten Ausführungsform weist die erste Durchgangsöffnung 11 ein Innengewinde auf. Mit diesem Innengewinde ist ein Außengewinde 16 des Verriegelungsstiels 12 verschraubbar. Hierzu kann der Verriegelungsstiel 12 an seinem proximalen Ende einen Werkzeugansatz (nicht dargestellt) aufweisen.

Der Verriegelungsstiel 12 weist ein als Steckkonus ausgebildetes distales Ende 17 auf, welches mit einer entsprechenden Aufnahme einer künstlichen Gelenkkugel verbindbar ist.

Der Sitz der Hülse 10 auf dem proximalen Ende 8 des Adapters 1 ist vorliegend noch besonders gesichert durch eine Schraube 18. Diese Schraube 18 greift durch den Verriegelungsstiel 12 durch eine darin vorgesehen zweite Durchgangsöffnung 19.

In Fig. 3 sind drei unterschiedliche Ausführungsformen für den Verriegelungsstiel 12 dargestellt. In Fig. 3a ist der Verriegelungsstiel 12 bis auf die zweite Durchgangsöffnung 19 massiv. Es findet also praktisch kein Einwachsen von Knochmaterial statt, weswegen diese Fixation die instabilste ist, dafür aber bei einem Revisionseingriff am leichtesten zu entfernen ist.

In Fig. 3b ist der Verriegelungsstiel 12 abschnittsweise mit einer offenmaschigen, dreidimensionalen Raumnetzstruktur 21 belegt, in welche und durch welche hindurch Knochentrabekel einwachsen und so für die stabilste Langzeitfixation sorgen.

In Fig. 3c schließlich ist der Verriegelungsstiel 12 zumindest teilweise als hohler Armierungskörper aus einem Gitternetzwerk 20 gebildet, in welches sich ebenfalls Knochenmaterial einorganisiert. Die Langzeitstabilität dieser Ausführungsform liegt zwischen jener der in Fig. 3a und Fig. 3b dargestellten Ausführungsformen.

### Bezugszeichenliste

- 1: Adapter
- 2: Stielteil
- 3: Femurstumpf
- 4: Raumnetzstruktur
- 5: distales Ende v. 2
- 6: Koppelungseinrichtung
- 7: exoprothetisches Standardteil
- 8: proximales Ende v. 1
- 9: Verriegelungsaufsatz
- 10: Hülse
- 11: erste Durchgangsöffnung
- 12: Verriegelungsstiel
- 13: Knochenkanal
- 14: Schenkelhals
- 15: Innengewinde
- 16: Außengewinde
- 17: distales Ende v. 12
- 18: Schraube
- 19: Durchgangsbohrung
- 20: Gitternetzwerk
- 21: Raumnetzstruktur
- 22: Buchse
- 23: Raumnetzstruktur

## Patentansprüche

1. Set zur Erstellung eines transkutanen Implantates, aufweisend
- einen intrakorporal mit seinem proximalen Stielteil (2) in einem Femurstumpf (3) verankerbaren Adapter (1) für ein exoprothetisches Standardteil (7), wobei das Stielteil (2) zumindest teilweise mit einer offenmaschigen dreidimensionalen Raumnetzstruktur (4) bedeckt ist und an seinem distalen Ende (5) mit einer Koppelungseinrichtung (6) für das exoprothetische Standardteil (7) versehen ist, und
- einen mit dem proximalen Ende (8) des Adapters (1) verbindbaren stielförmigen Aufsatz (10) zur Frakturstabilisation.

2. Set nach Anspruch 1, bei dem der Aufsatz ein von cranial neben dem Trochanter major einführbarer hülsenförmiger Armierungskörper ist.

3. Set nach Anspruch 1, bei dem der Aufsatz ein rohrförmiger Armierungskörper ist mit einem gekrümmten Verlauf und mit einer solchen Länge so ausgebildet ist, dass er von der Verbindungsstelle mit dem Adapter (1) im Femur (3) durch einen Knochenkanal in den Schenkelhals einführbar ist.

4. Set nach Anspruch 2 oder 3, bei dem der Armierungskörper aus einem zu einem Rohr geformten Gittemetzwerk besteht.

5. Set nach Anspruch 2 oder 3, bei dem der Armierungskörper aus körperverträglichem Metall besteht und zumindest abschnittsweise eine offenmaschige, dreidimensionale Raumnetzstruktur auf seiner Oberfläche aufweist.

6. Set zur Erstellung eines transkutanen Implantates, aufweisend
- einen intrakorporal mit seinem proximalen Stielteil (2) in einem Femurstumpf (3) verankerbaren Adapter (1) für ein exoprothetisches Standardteil (7), wobei das Stielteil (2) zumindest teilweise mit einer offenmaschigen dreidimensionalen Raumnetzstruktur (4) bedeckt ist und an seinem distalen Ende (5) mit einer Koppelungseinrichtung (6) für das exoprothetische Standardteil (7) versehen ist, und
- einen mit dem proximalen Ende (8) des Adapters (1) verbindbaren Verriegelungsaufsatz (9) zur Vorbereitung des Implantates als Gelenkkugelersatzimplantat.

7. Set nach Anspruch 6, bei dem der Verriegelungsaufsatz besteht aus
- einer von cranial neben dem Trochanter major einführbaren Hülse (10), die mit dem proximalen Ende (8) des Adapters (1) verbindbar ist, mit einer ersten Durchgangsöffnung (11), und
- einem Verriegelungsstiel (12), der in einen Knochenkanal (13) entlang des Schenkelhalses (14) des Femurs (3) einsetzbar ist, wobei der Verriegelungsstiel (12) durch die erste Durchgangsöffnung (11) in der Hülse (10) greift

8. Set nach Anspruch 7, bei dem die erste Durchgangsöffnung (11) der Hülse (10) ein Innengewinde (15) aufweist, mit dem ein Außengewinde (16) des Verriegelungsstiels (12) verschraubbar ist.

9. Set nach einem der Ansprüche 7 oder 8, bei dem das distale Ende (17) des Verriegelungsstiels (12) als konischer Steckzapfen ausgebildet ist.

10. Set nach einem der Ansprüche 7 bis 9, bei dem die von cranial in den Femurstumpf (3) einführbare Hülse (10) mit dem Adapter (1) mittels einer konischen Klemmverbindung verbindbar ist.

11. Set nach Anspruch 10, bei dem die konische Klemmverbindung durch eine Schraubverbindung zwischen einer die Hülse (10) durchsetzenden Schraube (18) und einer Gewindebohrung im Adapter (1) zusätzlich gesichert ist, wobei in dem Verriegelungsstiel (12) eine zweite Durchgangsbohrung (19) vorgesehen ist, durch welche die Schraube (18) greift.

12. Set nach einem der Ansprüche 7 bis 10, bei dem der Verriegelungsstiel (12) bis auf die zweite Durchgangsöffnung (19) massiv ist.

13. Set nach einem der Ansprüche 7 bis 11, bei dem der Verriegelungsstiel (12) wenigstens teilweise als hohler Armierungskörper aus einem Gitternetzwerk (20) gebildet ist.

14. Set nach einem der Ansprüche 7 bis 12, bei dem der Verriegelungsstiel (12) wenigstens bereichsweise mit einer offenmaschigen, dreidimensionalen Raumnetzstruktur (21) versehen ist.
